# EUROPEAN PATENT SPECIFICATION

(11) **EP 2 518 491 B1**
(45) Date of publication and mention of the grant of the patent: **23.10.2013**
(21) Application number: 11163619.7
(22) Date of filing: 25.04.2011
(51) Int. Cl.: G01N 33/24, G01N 35/02

(54) **In-Situ Soil Auto-Analyzer**
Automatisches in-situ Bodemanalysegerät
Analyseur automatique in-situ du sol

(43) Date of publication of application: 31.10.2012
(73) Proprietor: DizaynGrup Teknoloji Arastirma ve Gelistirme Ltd., 34220 Istanbul (TR)
(72) Inventor: Mirmahmutogullari, Ibrahim, 34220, Istanbul (TR); Gemici, Zafer, 34220, Istanbul (TR)
(74) Representative: Sevinç, Erkan

(56) References cited:
- US-A- 5 526 705
- M.S.ANDERSON, S.D.BRAYMAN: "The development and Operation of Instrumentation in a Mobile Laboratory for in situ, Real-time Screening and Characterization of Soils Using The Laser Ablation Sampling Technique", AMES LABORATORY (USDOE) ENVIRONMENTAL TECHNOLOGY DEVELOPMENT PROGRAM IOWA STATE UNIVERSITYFINAL REPORT AMES MOBILE LABORATORY PROJECT, IS-5115, 27 January 1995 (1995-01-27), pages 1-30, XP002659650,

## Description

### Technical Field of the Invention

The present invention relates to analyzing soil samples. More particularly, the present invention relates to an automated work station for analyzing a plurality of soil samples to determine characteristics of the soil samples such that obtained data is used to evaluate optimum field management in terms of micro and macro nutrient elements.

### Background of the Invention / Prior Art

In recent years, the securing of environmental preservation has been established as a precondition, and there is wide use of precision field management where the object is to reduce the application amount of agricultural materials, fertilizers, agricultural chemicals and other substances. In order to carry out such management, it is necessary to analyze the components of the soil, and in particular the carrying out of component analysis in real time is desired.

Crop management has grown rapidly in recent years. There are several components in the soil decisive in the overall performance in a given field with regard to a specific farm product. On the basis of the data obtained with respect to these components that are nutrients and micronutrients, what nutrients must be added to the soil to obtain optimal performance in the field can be determined with greater accuracy.

A problem which has been encountered with existing site specific crop management techniques is the slow processing or analyzing of soil samples taken from the field. Such processing and analyzing is typically done in order to determine certain attributes, both physical and chemical, of the soil samples obtained. Problems have also been encountered in the manipulation of the attributes, once determined, in order to decide which nutrients to apply to the field, and in actually applying the appropriate nutrients to the field.

Analysis of the soil samples has conventionally been extremely time consuming. Soil sample laboratories have conventionally been used in analyzing the soil samples to determine a number of nutrients and micronutrients found in the plants. A portion of the soil sample is taken to different sections of the sampling lab and analyzed for different parameters. Such a process is both time consuming and costly. Therefore, it has been inefficient to separately analyze large numbers of soil samples across a field to obtain a better understanding of the actual physical and chemical properties of the soil across the field.

A known device can be seen in US 5,526,705.

In order to increase the yield of agricultural products, it is necessary to replenish the deficient components in the soil such as organic components and fertilizers. On the other hand, when such fertilizer or the like is replenished beyond the required amount, there are cases where this is actually not good for the farm products.

### Objects of the Invention

One of the objects of the present invention is to provide an automated soil analysis work station in which a quantitative micro and macro nutrient element analysis can be carried out in situ.

A further object of the present invention is to provide a soil analysis system in which time duration for micro and macro nutrient element analysis procedure is substantially reduced.

A further object of the present invention is to provide a soil analysis system into which a drying system is incorporated such that results of micro and macro nutrient element analysis are obtained in substantially reduced time durations.

### Summary of the Invention

The soil auto-analyzer system according independent claim 1 is comprised of at least one pumping system, a soil cell, a sensor cell and a data logger. Said pumping system comprises a set of four injection means, each serving to the purpose of pumping a separate extraction solution. Said extraction solution in a given injection means is processed into said soil cell associated with a said given injection means on a given path. Each injection means on each analysis path according to the present invention is used to determine a separate attribute of the soil sample in the soil cell on an analysis path. The soil auto-analyzer system according to the present invention is equipped with a computer running several data analysis software, said computer being in communication with a central server associated with a database through a WAN.

### Brief Description of the Figures

Accompanying figures are given solely for the purpose of schematically exemplifying the concept of the present invention.

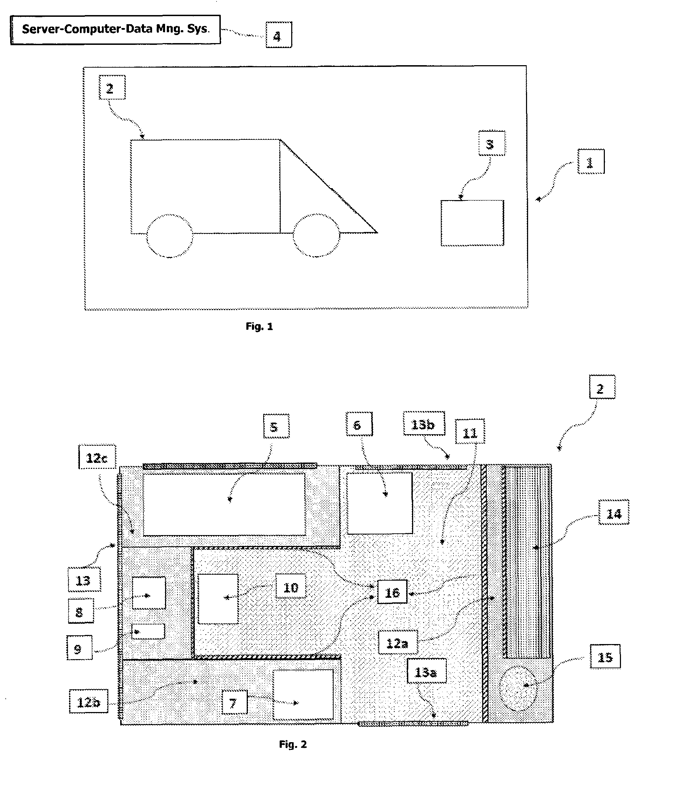

Figure 1 is a schematic demonstration of an in-situ soil auto-analyzer system according to the present invention.
Figure 2 is a schematic demonstration of a mobile laboratory of an in-situ soil auto-analyzer system according to the present invention.
Figure 3 is a schematic demonstration of the main components of the soil auto-analyzer system according to the present invention.
Figure 4 is a schematic demonstration of the drying system along with individual components in disassembled manner according to the present invention.
Figure 5a is a schematic demonstration of a soil cell body according to the present invention. Figure 5b is a schematic demonstration of a soil cell body in detail. Figure 5c is a schematic demonstration of a soil cell sample entrance and exit connector. Figure 5d is a schematic demonstration of a soil cell sample entrance plug.
Figure 6a is a schematic demonstration of a sensor cell body according to the present invention. Figure 6b is a schematic demonstration of a sensor cell body in detail. Figure 6c is a schematic demonstration of a sensor cell body entrance-exit connector.
Figure 7a is a schematic demonstration of a solid-state micro ion selective electrode according to the present invention. Figure 7b is a schematic demonstration of the active region of the solid-state micro ion selective electrode.
Figure 8 is a schematic demonstration of the data management system according to the present invention.

### Detailed Description of the Invention

The present invention will be explained with reference to the following part numbers as indicated in the drawings: Field (1), Mobile Laboratory (2), Farmer (3), Central Server and Database (4), Soil Auto-analyzer System (5), Quick Soil Drying System (6), Microwave Oven (7), Scale (8), Conductivity Meter (9), Mobile Laboratory Working Area (10), Laboratory Working Bench (11), Solution Preparation Bench (12a), Soil Sample Record Preparation Unit (12b), Side and Rear Entrance (13), Sample Reception Entrance (13a), Side Door (13b), Rear Door (13c), Upper Cabinets (14), Sink and Faucet (15), Lower Cabinets (16), Injector-type pump system (17), Plastic Injector (18), Soil Cell (19), Sensor Array (20), Data Logger (21), Flow Tubes (22), Electrical Connection (23), Computer (24), Printer (25), Print-out Report (26), Drier Body (27), Connection Part (28), Coarse Filter (29), First Container (30), Second Container (31), Thin Filter (32), Flow Path Entrance (33), Flow Path Exit (34), Soil Sample Entrance (35), Sample Receptacle (36), Connector (37), Sample Entrance Cap (38), O-ring (39), Micro Pore Filter (40), Filter Plug (41), Air Outlet Plug (42), Air Outlet (43), Sensor Slots (44), Connector Slots (45), Sensor Flow Path (46), Ion Selective Sensors (47), Reference Electrode (48), Connector (49), Extract Entrance (50), Extract Exit (51), Sensor Body (52), Conductor (53), Sensor Active Region (54), O-ring (55), Potentiometer Software (56), Fertilizer Software (57), Irrigation Software (58), Network (59) and representation of Data Flow (60), Extraction Solutions (61 to 64), Process paths (65 to 68).

The present invention proposes a system in which a quantitative micro and macro nutrient element analysis as to required fertilization can be carried out in situ and substantially reducing the analysis procedure in that respect. The soil auto-analyzer system (5) according to the present invention is equipped with a computer (24) running several data analysis software (56, 57, 58), said computer (24) being in communication with a central server associated with a database through a WAN.

In order for analyzing a soil sample in an efficient manner, a soil drying system (6) should be incorporated into the mobile laboratory (2) according to the present invention. Further a portable conductivity meter (9), a laboratory scale (8) and a microwave oven (7) are provided.

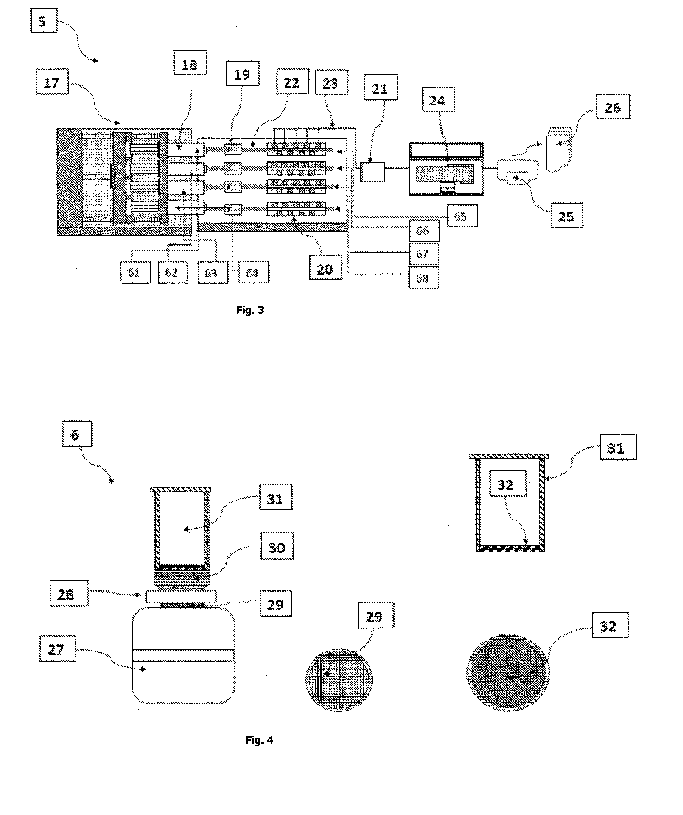

The soil auto-analyzer system according to the present invention is demonstrated in Fig. 3. It is comprised of at least one pumping system (17), a soil cell (19), a sensor cell (20) and a data logger (21). Said pumping system (17) comprises a set of four injection means (18), each serving to the purpose of pumping a separate extraction solution (61 to 64). Said pumping system (17) may either be configured to operate manually or automatically such that said extraction solution in a given injection means (18) is processed into said soil cell (19) associated with a said given injection means (18) on a given path (65 to 68). Said pumping system (17) is designed to calibrate amount and flow velocity of the extraction solution on each analysis path (65 to 68).

Each injection means (18) on each analysis path (65 to 68) according to the present invention is used to determine a separate attribute of the soil sample in the soil cell (19) on an analysis path (65-68). The extraction solution in said injection means (18) on said first analysis path (65) extracts macro nutrient elements from the soil sample in the soil cell (19) of the first path (65). The extraction solution on said second analysis path (66) extracts micro nutrient elements from the soil sample in the soil cell (19) on the second path (66). The extraction solution on the third analysis path (67) serves to determine lime requirement of the soil sample in the soil cell (19) on said path (67). Finally, the extraction solution on the fourth analysis path (68) serves to determine pH degree of the sample in the soil cell (19) of said fourth path (68). It is to be recognized that the number of analysis paths (65-68) can be modified to incorporate additional analysis paths with respective injection means (18), soil cells (19) and sensor cells (20).

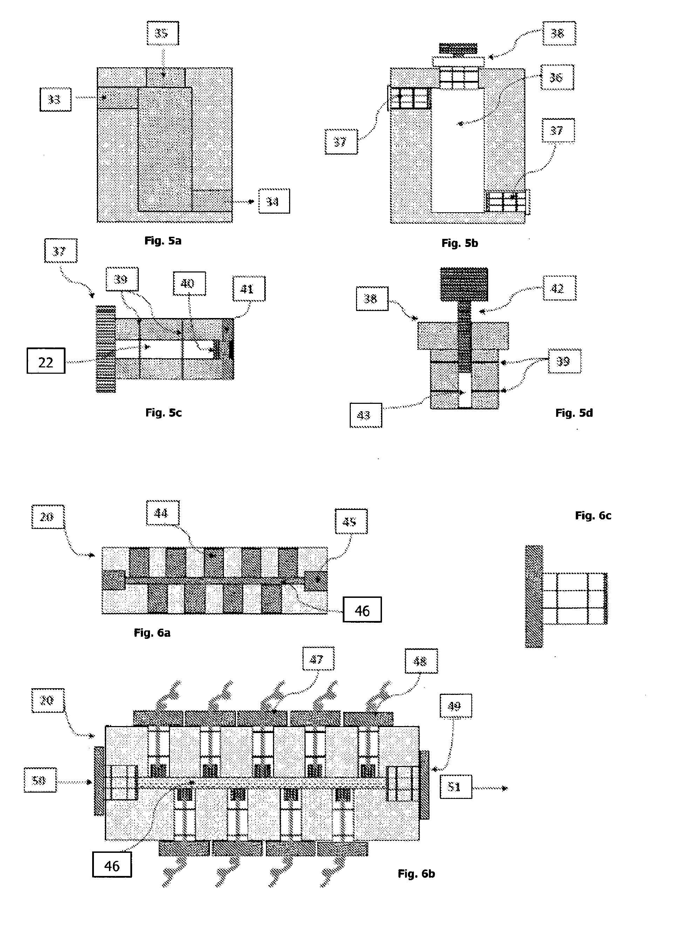

Extraction solutions (61-64) are conveyed to each respective soil cell (18) through tubes (22) with diameters in the range of micrometers. A continuous flow of the extraction solutions is ensured to provide extraction in each respective soil cell (19). Structure of a soil cell (19) according to the present invention is demonstrated in Fig. 5a to 5d. The extraction solution (61-64) enters said soil cell (19) through a flow path entrance (33) and leaves a sample receptacle (36) through a flow path exit (34). A separate entrance (35) is used to insert the samples. Said flow path entrance (33) and flow path exit (34) are formed by connectors (37) integrating said soils cells (19) into said analysis paths (65-68). The two connectors (37) of a soil cell (19) are connected to the injection means (18) and the sensor cells (20) through tubes (22). Said tubes are also preserved within said connector (37) bodies. Said in-connector (37) tubes (22) lead to a micro pore filter (40) and to a filter plug (41) securing said filter (40) in place. Said filter plug (41) has a central bore with a diameter corresponding to that of said flow tubes (22). This construction allows easy replaceability of worn-out filters (40).

According to the present invention, a sample entrance cap (38) firmly closes said sample receptacle (36). Further, an air outlet (43) provided within said sample entrance cap (38) allows removal of air in said sample receptacle (36). Said air outlet (43) is closed by a plug (43). A pair of o-rings (39) ensures impermeability. The time duration the extraction solution (61-64) interacts with the soil sample in said soil cell (19) can be controlled to continue a specified duration of five minutes.

After extraction process is terminated, extracts are then conveyed to said sensor cell (20) in order for initiating soil micro and macro nutrient analysis. A sensor cell (20) body is demonstrated in Fig. 6a, in which identical sensor slots (44) are shown. Sensor flow path (46) and sensor cell (20) entrance and exit connector slots (45) are also seen in Fig. 6a. Said sensor flow path (46) has a diameter in the range of millimeters and above.

Each sensor cell (20) according to the present invention, beside solid-state ion selective sensors (47), comprises a solid-state reference electrode (48) as shown in Fig. 6b. As is known to the person skilled in the art, a reference electrode's (48) potential does not vary in regard to different ions or concentrations thereof in the extract. Sensors (47) are secured in said sensor slots (45) in a removable manner, which thereby enables special configuration of a sensor cell (20).

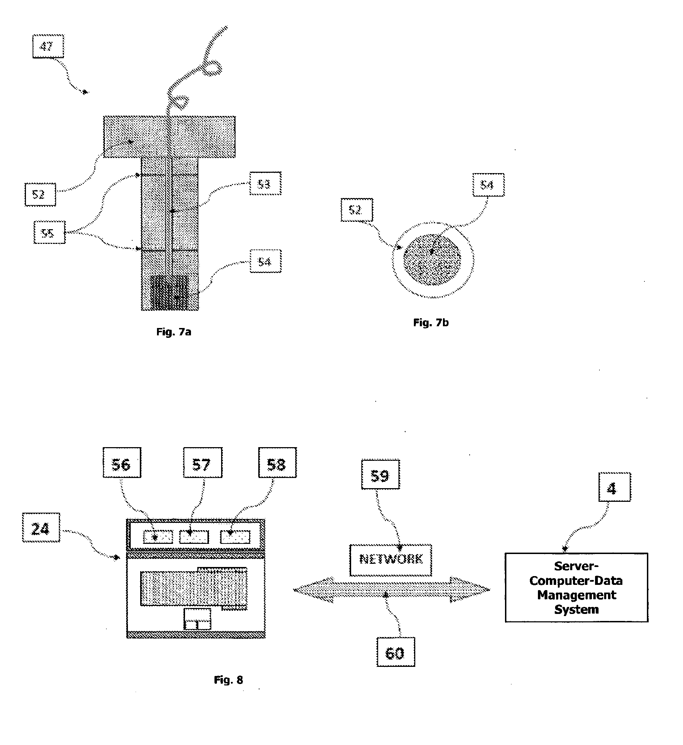

A solid-state ion selective electrode (47) is given in Fig. 7a. Sensor (47) body is made of a polymer-based material and electrical conduction is provided between an active region (54) of the sensor (47) and the data logger (21) by means of a conductive wire (53). A solid-state ion selective electrode (47) typically produces an electrical potential corresponding to the concentration of a specific ion in the extract.

Said conductive wire (53) is directly associated with said active region (54) of said sensor (47). The computer (24) in association with said data logger (21) according to the present invention enables monitoring, evaluation and storage of every analysis data carried out by the auto-analyzer system through a WAN. A potentiometric data analysis software (56) evaluates data received by said data logger's (21) potentiometric sensors and determines levels of macro and micro elements present in the soil sample.

In a nutshell, the present invention proposes a soil sample analyzing system comprising a soil cell (19) with a sample receptacle (36) for receiving a soil sample and an extraction solution (61-64). Said soil cell (18) is in fluid communication with a sensor cell (20). Said sensor cell (20) comprises a sensor flow path (46) and a plurality of solid-state ion selective sensors (47) having active regions (54) in communication with said sensor flow path (46). Said active regions (54) are in electrical conduction communication with a data logger (21).

The in-situ analysis system according to the present invention can be used to create a soil map as to obtained soil properties from. A soil map correlated with the soil properties at each physical position is created; the soil map can be effectively utilized for future field management future. The position information can be calculated by using GPS.

The data stored in in-situ analysis system according to the present invention can be utilized by means of a peripheral of said computer (24), in the form of a recording medium such as a CD-ROM, DVD or the like on which such data is recorded, reading out such data by said computer (24), and then storing such data in the storage portion inside said computer (24). Further, a drive device such as a CD-ROM drive or the like which reads data into a recording medium may be prepared, and then by setting a recording medium into the drive device, various information can be read onto the recording medium inside the drive device at such time.

As discussed earlier, it is possible to transmit the above-described data via the Internet or another network.

Alternatively, said extraction solution (61-64) according to the present invention can continuously be processed through said soil cell (19) in a repetitive manner by means of a closed loop path. In this configuration, said pumping system (17) processes said extraction solution (61-64) through a closed loop flow path on a certain part of which said soil cell (19) is located, the extraction solution being then pumped to the sensor cell (20) through a second pumping system.

## Claims

1. A soil sample analyzing work station comprising a soil cell (19) with a sample receptacle (36) for receiving a soil sample and an extraction solution (61-64), said soil cell (19) being in fluid communication with a sensor cell (20), said sensor cell (20) comprising a sensor flow path (46) and a plurality of solid-state ion selective sensors (47) having active regions (54) in communication with said sensor flow path (46), said active regions (54) being in electrical conduction communication with a data logger (21) **characterized in tha**t**,**
said sensor cells (20) comprise a plurality of sensor slots (45) in which said sensors (47) are secured in a removable manner,
said station comprises a pumping system (17) that comprises at least one injection means (18) pumping said extraction solution (61 to 64),
said pumping system (17) processes said extraction solution in an injection means (18) into said soil cell (19) associated with said injection means (18) on a path (65 to 68) and,
said pumping system (17) is designed to calibrate amount and flow velocity of the extraction solution on said analysis path (65 to 68).

2. A soil sample analyzing work station as set forth in Claim 1 wherein said station comprises a soil drying system (6).

3. A soil sample analyzing work station as set forth in Claim 1 wherein said station is equipped with a computer (24) running data analysis software (56, 57, 58), said computer (24) being in communication with a central server associated with a database through a WAN.

4. A soil sample analyzing work station as set forth in Claim 1 wherein said extraction solution is continuously processed through said soil cell (19) in a repetitive manner by means of a closed loop path.

5. A method for analyzing a soil sample using the work station according to Claim 1 comprising the steps of applying an extraction solution to said soil sample, processing obtained extract to a sensor cell (20), determining macro and micro elements in said soil sample, processing information as to amounts of macro and micro elements in the soil sample to determine deficient components in the form of organic components and fertilizers, storing said information as to deficient soil components in relation to a geographic location in a database.

## Patentansprüche

1. Eine Arbeitsstation zur Analyse von Erdproben, umfassend eine Erdzelle (19) mit einem Probenbehälter (36) zur Aufnahme einer Erdprobe und einer Extraktionslösung (61-64), wobei die Erdzelle (19) in flüssiger Kommunikation mit einer Sensorzelle (20) ist, wobei die Sensorzelle (20) einen Sensorströmungsweg (46) und eine Vielzahl von für Festkörperionen selektive Sensoren (47) mit aktiven Bereichen (54) umfasst, die in Kommunikation mit dem Sensorströmungsweg (46) sind, wobei die aktiven Bereiche (54) in elektrischer Leitungskommunikation mit einem Datenlogger (21) sind,
**dadurch gekennzeichnet, dass**
- die Sensorzellen (20) eine Vielzahl von Sensorschlitzen (45) umfassen, in welchen die Sensoren (47) in einer entfernbaren Weise gesichert werden,
- die Station ein Pumpensystem (17) umfasst, das mindestens ein Injizierungsmittel (18) umfasst, das die Extraktionslösung (61-64) pumpt,
- das Pumpensystem (17) die Extraktionslösung in einem Injizierungsmittel (18) in die Erdzelle (19), die mit dem Injizierungsmittel (18) assoziiert ist, auf einem Pfad (65-68) einführt und,
- das Pumpensystem (17) gestaltet ist, um die Menge und die Fließgeschwindigkeit der Extraktionslösung in dem Analysierungspfad (65-68) zu kalibrieren.

2. Arbeitsstation zur Analyse einer Bodenprobe gemäß Anspruch 1, wobei die Station ein System (6) zum Trocknen der Erde umfasst.

3. Arbeitsstation zur Analyse einer Bodenprobe gemäß Anspruch 1, wobei die Station ausgestattet ist mit einem Computer (24), der die Software für die Datenanalyse (56, 57, 58) führt, wobei der Computer (24) mit einem zentralen Server, der mit einer Datenbank assoziiert ist, in Kommunikation durch ein WAN ist.

4. Arbeitsstation zur Analyse einer Bodenprobe gemäß Anspruch 1, wobei die Extraktionslösung kontinuierlich durch die Erdzelle (19) in einer repetitiven Weise mittels eines geschlossenen Schleifenpfades geführt wird.

5. Ein Verfahren zur Analyse einer Erdprobe unter Verwendung der Arbeitsstation gemäß Anspruch 1, umfassend die Schritte des Auftragens einer Extraktionslösung in eine Bodenprobe, Einführen des erhaltenen Extraktes in eine Sensorzelle (20), Bestimmen der Makro- und Mikroelemente in der Bodenprobe, Verarbeiten der Informationen bezüglich der Mengen von Makro- und Mikroelementen in der Bodenprobe, um unzureichende Komponenten in der Form von organischen Komponenten und Düngemittel zu bestimmen, Speichern der Information betreffend die unzureichenden Bodenkomponenten in Bezug auf einen geografischen Standort in einer Datenbank.

## Revendications

1. Station de travail pour analyse d'échantillon de sol, comprenant une cellule de sol (19) avec un logement d'échantillon (36) pour recevoir un échantillon de sol et une solution d'extraction (61 à 64), ladite cellule de sol (19) étant en communication fluidique avec une cellule de capteur (20), ladite cellule de capteur (20) comprenant un trajet de capteur d'écoulement (46) et une pluralité de capteurs à sélection ionique à semi-conducteur (47) ayant des régions actives (54) en communication avec ledit trajet de capteur d'écoulement (46), lesdites régions actives (54) étant en communication par conduction électrique avec un enregistreur de données (21), **caractérisée en ce que**
lesdites cellules de capteur (20) comprennent une pluralité de fentes de capteur (45) dans lesquelles lesdits capteurs (47) sont fixés de manière amovible,
ladite station comprend un système de pompage (17) qui comprend au moins des moyens d'injection (18) pompant ladite solution d'extraction (61 à 64),
ledit système de pompage (17) traite ladite solution d'extraction dans des moyens d'injection (18) dans ladite cellule de sol (19) associée auxdits moyens d'injection (18) sur un trajet (65 à 68) et,
ledit système de pompage (17) est conçu pour calibrer une quantité et une vitesse d'écoulement de la solution d'extraction sur ledit trajet d'analyse (65 à 68).

2. Station de travail pour analyse d'échantillon de sol selon la revendication 1, ladite station comprenant un système de séchage de sol (6).

3. Station de travail pour analyse d'échantillon de sol selon la revendication 1, ladite station étant équipée d'un ordinateur (24) exécutant un logiciel d'analyse de données (56, 57, 58), ledit ordinateur (24) étant en communication avec un serveur central associé à une base de données par l'intermédiaire d'un WAN.

4. Station de travail pour analyse d'échantillon de sol selon la revendication 1, dans laquelle ladite solution d'extraction est traitée en continu par ladite cellule de sol (19) de manière répétitive au moyen d'un trajet en boucle fermée.

5. Procédé pour analyser un échantillon de sol utilisant la station de travail selon la revendication 1, comprenant les étapes d'application d'une solution d'extraction audit échantillon de sol, de traitement de l'extrait obtenu vers une cellule de capteur (20), de détermination de macro- et de micro-éléments dans ledit échantillon de sol, de traitement d'informations concernant des quantités de macro- et de micro-éléments dans l'échantillon de sol pour déterminer des composants déficients sous la forme de composants organiques et de fertilisants, de stockage desdites informations concernant des composants de sol déficients relativement à un emplacement géographique dans une base de données.
